Europäisches Patentamt

(19) European Patent Office

Office européen des brevets

(11) Publication number : **0 109 441 B1**

## (12) EUROPEAN PATENT SPECIFICATION

(45) Date of publication of patent specification :
17.11.94 Bulletin 94/46

(51) Int. Cl.⁵ : **G01N 33/53**

(21) Application number : 83902157.3

(22) Date of filing : 20.05.83

(86) International application number :
PCT/US83/00781

(87) International publication number :
WO 83/04313 08.12.83 Gazette 83/28

## (54) HUMAN-HUMAN HYBRIDOMAS FOR NEOPLASMS.

(30) Priority : 21.05.82 JP 84843/82

(43) Date of publication of application :
30.05.84 Bulletin 84/22

(45) Publication of the grant of the patent :
17.11.94 Bulletin 94/46

(84) Designated Contracting States :
AT BE CH DE FR GB LI LU NL SE

(56) References cited :
EP-A- 44 722
GB-A- 2 086 937
PROCEEDINGS OF THE NATL. ACADEMY OF
SCIENCES USA, vol. 77, no. 11, November
1980; SCHLOM et al., pp. 6841-6845
NATURE, vol. 244, 17 August 1973;
SCHWABER et al., pp. 444-447
CLINICAL CHEMISTRY, vol. 27, no. 9, September 1981; BROWN et al., pp. 1592-1596
FEDERATION PROCEEDINGS, vol. 41, no. 3, 01
March 1982; GLASSY et al., p. 553, no. 1657
PROCEEDINGS OF THE NATL. ACADEMY OF
SCIENCES USA, vol. 77, no. 8, August 1980;
GILLILAND et al., pp. 4539-4543

(73) Proprietor : THE REGENTS OF THE
UNIVERSITY OF CALIFORNIA
300 Lakeside Drive,
22nd Floor
Oakland, California 94612-3550 (US)

(72) Inventor : HANDLEY, Harold H.
2447 Newcastle
Cardiff-By-The-Sea, CA 92007 (US)
Inventor : GLASSY, Mark C.
10246 Parkdale
San Diego, CA 92126 (US)
Inventor : HAGIWARA, Hideaki
Riverside Manshion No. 806
1-3-4 Shinkitano
Yodogawa-ku Osaka-shi (JP)
Inventor : HAGIWARA, Yoshihide
4-14, Hirai Sanso
Takarazuka-shi
Hyogo-ken (JP)

(74) Representative : Haigh, Charles Roy et al
J.A. KEMP & CO.
14 South Square
Gray's Inn
London WC1R 5LX (GB)

## Description

### Field of the Invention

The mammalian immune system has a matchless ability to produce molecules with specificity and avidity for a particular spatial and polar structure, as may be found with sequences of amino acids and sugars. For a long period of time, one was dependent upon producing antibodies employing the immune system in vivo. The resulting polyclonal antibodies demonstrated high specificity for a specific antigen, but could not discriminate between various sites on the antigen and, furthermore, were a mixture of antibodies of varying specificity and avidity. Thus, one observed the averaging over the entire composition and not the properties of a specific antibody.

With the seminal discovery by Milstein and Kohlen one can now produce homogeneous compositions of antibodies by fusing a B-lymphocyte with a myeloma cell to produce a cell referred to as a hybridoma. For the most part, the use of this technology has been limited to mouse cells, where stable myeloma lines have served as fusion partners to provide stable hybridomas which can be produced with high efficiency and are capable of being maintained as productive entities over long periods of time. Higher organisms, particularly humans, have proven to be much more intractable in developing fusion partners and hybridomas. However, in 1980, the first human fusion partner was reported by Drs. Olsson and Kaplan and since that time, an additional few human fusion partners have been reported. Nevertheless, the preparation of hybridomas by human-human crosses nas remained difficult due to problems of efficiency in fusion, culturing the cells, and maintaining their productive capabilities. However, because of the many. advantages of having human hybridomas which produce antibodies allogenic to a human host, particularly for in vivo applications, human hybridomas remain of great interest. In other instances, even with the difficulties encountered with human-human crosses, the human hybridoma may be preferable to a heterogeneic cross, where the resulting hubridoma may lose the genetic information for the monoclonal antibodies after a number of passages.

One of the areas of interest for the use of monoclonal antibodies is in diagnosing and treating cancer. Monoclonal antibodies for these purposes desirably are specific for a particular type of cancer or subset of cancers, rather than being specific for a particular host cancer cell. it is therefore desirable to develop monoclonal antibodies which can be used in the diagnosis and treatment of human cancers.

### Description of the Prior Art

Nowinski et al., Science (1980) 210:537-539 describe human monoclonal antibodies against Forssman antigen. Corce et al., Nature (1980) 288:488-489 describe human hybridomas secreting antibodies to measles virus. Olsson and Kaplan, PNAS USA (1980) 77:5429-5431 describe human-human hybridomas producing monoclonal antibodies of predefined antigenic specificity as well as the fusion partner employed for production of the antibodies. See also copending application Serial No. 247,652, filed March 26, 1981.

Schlom, PNAS USA (1980) 77:6841-6845 describes monoclonal antibodies for breast cancer and Sikora, Brit. J. of Cancer (1981) 43:696 describes separating in situ lymphocytes from a cancer providing antibodies specific for the cancer. In the Proceedings of the 15th Leukocyte Culture Conference, Parker and O'Brien, eds., Wiley Interscience, N.Y., Dec. 5-10, 1982, the subject hybridoma is described. This abstract is incorporated herein by reference.

GB-A-2 086 937 describes the production of human monoclonal antibodies from a hybridoma obtained by fusing a human myeloma cell with a human lymphocyte obtained from a patient contaminated with an active virus such as rabies, influenza, measles, vaccinia, polio, etc. There is no suggestion in this document that it would be possible to secure a monoclonal antibody of specificity such that it could distinguish neoplastic cells from human non-malignant cells.

### SUMMARY OF THE INVENTION

The present invention provides human hybridomas capable of producing human monoclonal antibodies which distinguish human neoplastic cells from normal human cells, wherein said human hybridoma is obtainable by fusion of a B-lymphocyte from a draining lymph node of a human host having a neoplasia with a fusion partner for human cells, wherein said fusion partner is a human immortalized B-cell which itself does not secrete immunoglobulins; or human hybridomas derived therefrom. The present invention also includes human monoclonal antibodies obtained from the hybridomas of the invention.

In accordance with a further aspect of the present invention, the antibodies of the invention, or fragments thereof, are used to determine the presence of a neoplasm by combining a sample from a host suspected of having a neoplasm with the monoclonal antibodies or fragments thereof followed by the detecting of the presence of the binding of the monoclonal antibodies or fragments thereof.

According to a still further aspect of the present invention, there is provided a method for producing the human monoclonal antibodies of the invention by growing the hybridoma of the invention and recovering the antibodies produced by the hybridoma. Where necessary, these antibodies can be labeled.

In accordance with a still further aspect of the present invention, there is provided human monoclonal antibodies or fragments thereof, as described in the immediately preceding three paragraphs, for use in a method of treatment of the human body by therapy.

## DESCRIPTION OF SPECIFIC EMBODIMENTS

Human monoclonal antibodies specific for neoplastic cells from solid tumors are obtained from human x human fusions employing B lymphocytes, e.g., from lymph nodes draining a solid tumor. Particularly, lymph nodes are selected which appear to be active based on necrosis of tumor cells in the vicinity of the lymph node in an immunocompetent host.

The draining lymph node(s) may be isolated in conjunction with a variety of human tissue, e.g., cervix, mammary, colon, lungs, prostate, skin, etc. Of particular interest are lymph nodes from the spinal area.

The fusion partner may be any convenient immortalized B-cell, which does not secrete immunoglobulins, individual chains or fragments thereof, can be selected against, as with HAT medium, and desirably has a high fusion efficiency. Illustrative fusion partners are UC729-6, J-4 (SKO-007), and GM1500 6TG-A12.

The fusions may be performed as described in the literature employing PEG1500 as fusogen, plating the cells in HAT medium in a plurality of wells and then screening supernatants in the viable cell wells for antibodies of interest. Wells positive for reactivity are then cloned by limiting dilution and expanded.

Of particular interest are the novel hybridomas CLNH5 and CLNH11, hybridomas obtained from CLNH5 and 11, antibodies derived from such hybridomas, derivatives of such antibodies and the use of the antibodies and their derivatives for diagnosis and therapy. CLNH5 and 11 are obtained by fusion between the fusion partner UC729-6 with lymphocytes from lymph node cells of a patient having cervical cancer. UC729-6 was deposited on 25 March 1981 at the A.T.C.C. Maryland, USA, with Accession No. CRL 8061.

For the production of CLNH5 and CLNH11, the lymphocytes employed for fusion were from a draining lymph node from the spinal area and peripheral blood lymphocytes from a patient having cervical carcinoma. The fusion is performed by combining the patient's lymphocytes from the lymph node with the fusion partner UC729-6 at a ratio of about 2:1 in a solution of about 35% polyethylene glycol in HEPES buffered RPMI 1640. The mixture of cells is then suspended in appropriate selective medium, particularly HAT medium containing about 10% fetal bovine serum, placed in wells at about $10^5$ cells per well and a sufficient time permitted for the cells to grow. The selective medium is replaced from time to time.

Wells from the above fusion provided clones specifically reactive with the cervical cancer cells of the host patient which were designated CLNH5 and 11. These wells provided human IgM and IgG monoclonal antibodies, respectively, which react with antigen found on a variety of cervical carcinomas and other tumor cell lines, e.g., small cell carcinoma of the lung, but not with normal tissues and normal cell lines, which were tested.

The hybridomas and monoclonal antibodies can find use in a variety of ways, particularly as sources of genetic material, as reagents, and as precursors to products which find use as reagents.

The subject hybridomas may be used as a source for genetic material. For example, the subject hybridomas may be fused with other fusion partners to provide novel hybridomas having the same secretory capabilities as CLNH5 and 11 to provide antibodies having the same specificity. Such fusions may result in the production of antibodies having different heavy chains so as to provide the other classes or subclasses of antibodies, e.g., G, A or M.

The hybridoma may also be used as a source of DNA, which by hybrid DNA technology, the genes may be excised, introduced into a lymphoma for production of the mature antibodies.

The monoclonal antibodies can be used in a variety of ways, both in _vivo_ and in _vitro_ diagnosis, as well as in therapy. For many applications, the antibodies will be labeled with a compound which imparts a desired property to the antibodies, such as providing a detectable signal, providing cytotoxicity, providing for localized electromagnetic radiation, or the like. Labels may include radionuclides, enzymes, fluorescers, toxins or the cytotoxic fragment of toxins, particles, metals, metalloids, etc. The antibodies may be incorporated in liposome membranes or modified with lipids, so as to be incorporated in such membranes. The antibodies by themselves or labeled, may be used in _in vitro_ diagnosis for measuring the presence of antigens associated with a neoplasm such as cervical cancer, for in _vivo_ diagnosis for introduction into a host, e.g., intravenously, in a physiologically acceptable carrier, e.g., PBS, or may be introduced for therapeutic purposes in the same manner.

The antibodies by themselves or labeled, may also be used for treating a neoplasm in human host such as cervical carcinoma, prostate tumor, colon carcinoma, lung cancer, breast cancer and melanoma. The antibodies of this invention are easily soluble in physiological saline, and therefore can be injected intravenously or intramuscularly as a saline solution or a drip. Furthermore, the antibodies of the invention can be used in the form of an ointment or suppository.

The amount of antibody employed will vary depending upon the particular application. Introduction of antibodies for diagnostic and therapeutic purposes has been extensively described in literature.

The entire antibody need not be used, for many applications only a fragment having intact variable regions will suffice. For example, Fab fragments, $F(ab')_2$ fragments, or Fv fragments may suffice. Other preferred embodiements of the invention are the subject matter of subclaims.

The following examples are offered by way of illustration and not by way of limitation.

EXPERIMENTAL MATERIALS AND METHODS

Fusion and Selection of Hybridomas.

Lymph nodes were teased with nugent forceps in RPMI 1640 media and isolated lymphocytes were cultured overnight at 37°C and 5% $CO_2$ in RPMI 1640 with 10% fetal calf serum (FCS) and 2mM L-glutamine. Lymphocytes were counted and mixed at a ratio of 2:1 with the human lymphoblastoid B cell line UC729-6 (Handley and Royston. 1982, in Hybridomas in Cancer Diagnosis and Treatment, eds. Mitchell and Oettgen, pp. 125-132, Raven Press, N.Y.), then fused with polyethylene glycol 1500 by a modification of the technique by Gefter et al., Somatic Cell Genetics (1977) 3:321-336. Fused cells were plated at $10^5$ cells/well in a Costar 96 well microtiter plate with Hypoxanthine-Amethopterin-Thymidine (HAT, Littlefield, Science (1964) 145:709-710) supplemented RPMI 1640 with 10% FCS and L-glutamine. Within 10-20 days, wells positive for hybridoma growth were assayed for human antibody production and their reactivity to a limited human cell panel by an enzyme immunoassay (EIA). Wells positive for reactivity were cloned by limiting dilution without the use of feeder layers and expanded for further study.

Enzyme Immunoassay.

Human MoAbs and their reactivity to cells were detected by a modification by an EIA previously described (Handley et al. J. of Immunologic Methods (1982) 54:291-296, as modified by Glassy et al., J. Immunologic Methods (1983) in press). Briefly, 50µl of either an affinity purified goat anti-human Ig or a $4 \times 10^6$ target cell/ml suspension was immobilized in triplicate wells of an immunofiltration manifold. (The specifically designed microtiter plate which serves as both an incubation chamber and filtration manifold (VP no. 107; V and P Scientific, San Diego, CA). The bottom of each well contains a 0.6mm hole over which is placed a 6mm diameter glass fiber filter. Surface tension prevents fluid volumes less than 100µl from draining through the hole until a vacuum is applied. When vacuum is applied, fluid is drawn through the filter and out the drain hole leaving particulate matter trapped on the filter. After washing 3x with 0.3% gelatin in phosphate buffered saline, 50µl of hybridoma supernatant were incubated 30 min at room temperature. Filters were then washed and incubated with 50µl of a horseradish peroxidase conjugated goat anti-human Ig for an additional 30 min. Filters were washed again and incubated with 150µl of a 400 µg/ml solution of ortho-phenylene diamine in citrate buffer. 100µl of each well were then transferred to a new plate containing 50µl of 2.5M $H_2SO_4$ and read on a Dynatek (Alexandria, VA) MR 580 micro-ELISA reader at 492nm.

Hybridoma culture fluids were precipitated with 50% ammonium sulfate and crude Ig fractions collected. The precipitates were dissolved in phusiological saline and purified by affinity chromatography using S-aureus Protein A-bound Sepharose with IgC and Sepharose-(sheep anti(humanIgM) antibody) with IgM. From 1L of the culture fluid of CLNH5, 2.2mg IgM was obtained, while from 1L of the culture fluid of CLNH11, 3.0mg IgG was obtained.

RESULTS

Table 1 outlines the results of the fusion attempting to produce anti-SCCC (squamous cell carcinoma of cervix) human MoAbs. The fusion producing CLNH5 and CLNH11, human-human hybridomas secreting a MoIgMk and a MoIgG reactive with SCCC cell lines, generated 6 growth positive wells of 80 wells plated. Hybridomas CLNH5 and CLNH11 were cloned and expanded when found to react with the cervical carcinoma cell lines, CaSki and Hela.

## TABLE 1

### GENERATION AND IDENTIFICATION OF HUMAN MoAbs

| Lymph Node draining | #Lympho-cytes fused | #Hybri-domas generated | #Secret-ing M | | G | A | #Human reactive |
|---|---|---|---|---|---|---|---|
| Cervical Carcinoma (SCCC) | $7.0 \times 10^6$ | 6 | 2 | | 1 | 0 | 2 (CLNH5 and 11) |

The relative amounts of human MoAb bound to each of the cell lines listed was measured by EIA.

Antibody (IgM) secreted by CLNH5 shows positive reactivity with carcinomas of the cervix (CaSki, Hela), lung (T293, Calu-1, and SK-MES-1), melanoma (SK-MEL-28), and prostate (LnCap) and was negative for normal fibroblasts, T lymphocytes and peripheral blood lymphocytes. Antibody (IgG) secreted by CLNH11 shows positive reactivity with carcinomas of the cervix (CaSki, Hela), prostate (PC-3), breast (ZR-76-1), colon (COLO-205) and melanoma (G-361) and was negative for normal fibroblasts (WI-38 and MRC-9), T. lymphocytes and peripheral blood lymphocytes.

The cytobiochemical properties of the hybridomas of the present invention are shown below.

Hybridoma CLNH5:-

(1) Number of chromosomes: 60 to 90 (maximum frequency 80).
(2) It secretes human immunoglobulin M (IgM).
(3) Doubling time: 30-40 hours.
(4) Lymphocytic single cell.
(5) Its DNA content is at least two times, for example, 2 to 2.5 times, that of normal human lymphocytes.
(6) IgM binds to human cervical carcinoma cells and the other carcinomas mentioned above.

In addition, the above hybridoma CLNH5 can be proliferated in HAT medium (medium containing hypoxanthine, amethopterin and thymidine).

Hybridoma CLNH11:-

(1) Number of chromosomes: 60 to 90 (maximum frequency 80).
(2) It secretes human immunoglobulins G (IgG).
(3) Doubling time: 30-40 hours.
(4) Lymphocytic single cell.
(5) Its DNA content is at least two times, for example 2 to 2.5 times, that of normal human lymphocytes.
(6) IgG binds to human cervical carcinoma cells, and the other carcinomas mentioned above.

In addition, the above hybridoma CLNH11 can be proliferated in HAT medium.

The relative DNA content (the ratio to the DNA content of normal human lymphocytes) was determined by a method which comprises dyeing the hybridoma and then separating and analyzing it by a cytofluorometer.

The properties of the monoclonal human immunoglobulines in accordance with this invention are shown below.

Monoclonal Human Immunoglobulin Produced by the Hybridoma CLNH5

(a) It is human immunoglobulin M (IgM).
(b) It has a stronger binding affinity to cell lines, Hela and CaSki, than to normal fibroblasts (WI-38).
(c) It does not react with human red blood cells, nor shows an agglutination reaction on human red blood cells.
(d) It is composed of heavy chains (H chains) and light chains (L chains), has a molecular weight of about 180,000 (monomer), and exists as a pentamer in the culture fluid.

Monoclonal Human Immunoglobulin Produced by the Hybridoma CLNH11

(a) It is human immunoglobulin G (IgG).

(b) It has a stronger affinity to cell lines, Hela and CaSki, than to normal fibroblasts (WI-38).

(c) It does not react with human red blood cells, nor shows an agglutination reaction on human red blood cells.

(d) It is composed of H chains and L chains and has a molecular weight of about 150,000.

The binding activity of human monoclonal antibodies distinguishing neoplastic cells from normal cells was measured as follows:

An original human tissue section including carcinoma cells and normal cells was fixed on a glass plate by glutaraldehyde, and then stained by enzyme immunoassay according to the method of Sternberger et al. J. Hist. Cyto. 18 315 (1970).

The subject monoclonal antibodies are useful for the diagnosing, imaging and potentially for treating cervical carcinoma as well as other reactive tumors. Because of the specificity of the monoclonal antibodies over a rnage of cervical carcinomas from different hosts, the subject antibodies can be used in different hosts, rather than solely with the host source of the antigen. Because the subject antibodies are human, they are less likely to produce a significant immune response when employed in in vivo diagnosis or therapy.

## Claims

### Claims for the following Contracting States : BE, CH, DE, FR, GB, LU, NL, SE

1. Human hybridomas capable of producing human monoclonal antibodies which distinguish human neoplastic cells from normal human cells, wherein said human hybridoma is obtainable by fusion of a B-lymphocyte from a draining lymph node of a human host having a neoplasia with a fusion partner for human cells, wherein said fusion partner is a human immortalized B-cell which itself does not secrete immunoglobulins, or human hybridomas derived therefrom.

2. Human hybridomas CLNH5 or CLNH11 according to claim 1.

3. Human monoclonal antibodies obtained from human hybridomas according to one of claims 1 or 2.

4. Human monoclonal antibodies according to claim 3 wherein the neoplastic cells are solid tumor cells.

5. Human monoclonal antibodies according to claim 3, wherein the neoplastic cells are prostate carcinoma cells, lung small cell carcinoma cells, cervical carcinoma cells, colon carcinoma cells or melanoma cells.

6. Human monoclonal antibodies according to claim 3 wherein the neoplastic cells are cervical carcinoma cells.

7. Human monoclonal antibodies according to any one of claims 3 to 6 or fragments thereof labeled with a label capable of providing a detectable signal.

8. Human monoclonal antibodies according to claim 7 or fragments thereof, wherein said label is a radionuclide.

9. Human monoclonal antibodies according to any one of claims 3 to 6 or fragments thereof, labeled with a toxin.

10. Use of the monoclonal antibodies or fragments thereof according to any one of claims 3 to 9 to determine the presence of a neoplasm which comprises:
    - combining a sample from a host suspected of having a neoplasm with the monoclonal antibodies or fragments thereof and
    - detecting the presence of the binding of said monoclonal antibodies or fragments thereof.

11. The use of the monoclonal antibodies according to claim 10, wherein said sample is host tissue.

12. The use of the monoclonal antibodies according to claim 10 or 11, wherein said neoplasm is a solid tumor.

13. The use of the monoclonal antibodies according to claim 12, wherein said solid tumor is a cervical carcinoma, prostate tumor, colon carcinoma, lung cancer, breast cancer or melanoma.

14. A method for producing human monoclonal antibodies as defined in any one of claim 3 to 9, which comprises growing a hybridoma as claimed in claim 1 or 2 and recovering the antibodies produced by the hybridoma and, where desired, labeling the antibodies.

15. Human monoclonal antibodies or fragments thereof according to any one of claims 3 to 9 for use in a method of treatment of the human body by therapy.

**Claims for the following Contracting State : AT**

1. A method for producing human monoclonal antibodies, which can distinguish human neoplastic cells from human normal cells, which comprises growing a hybridoma and recovering the antibodies produced by the hybridoma wherein the hybridoma is obtainable by fusion of a B-lympocyte from a draining lymph node of a human host having a neoplasia with a fusion partner for human cells, wherein said fusion partner is a human immortalized B-cell which itself does not secrete immunoglobulins.

2. A method according to claim 1 wherein the hybridoma is CLNH5 or CLNH11.

3. A method according to claim 1 or 2 wherein the neoplastic cells are solid tumor cells.

4. A method according to claim 1 or 2 wherein the neoplastic cells are prostate carcinoma cells, lung small cell carcinoma cells, cervical carcinoma cells, colon carcinoma cells or melanoma cells.

5. A method according to claim 1 or 2 wherein the neoplastic cells are cervical carcinoma cells.

6. A method according to any one of the preceding claims wherein the antibodies or fragments thereof are labelled with a label capable of providing a detectable signal.

7. A method according to claim 6 wherein the label is a radionuclide.

8. A method according to claim 6 wherein the label is a toxin.

9. Use of the monoclonal antibodies or fragments thereof according to any one of claims 3 to 8 to determine the presence of a neoplasm which comprises:
   - combining a sample from a host suspected of having a neoplasm with the monoclonal antibodies or fragments thereof and
   - detecting the presence of the binding of said monoclonal antibodies or fragments thereof.

10. The use of the monoclonal antibodies according to claim 9, wherein said sample is host tissue.

11. The use of the monoclonal antibodies according to claim 9 or 10, wherein said neoplasm is a solid tumor.

12. The use of the monoclonal antibodies according to claim 11, wherein said solid tumor is a cervical carcinoma, prostate tumor, colon carcinoma, lung cancer, breast cancer or melanoma.

13. Human monoclonal antibodies or fragments thereof obtained by a process according to any one of claims 1 to 8 for use in a method of treatment of the human body by therapy.

**Patentansprüche**

**Patentansprüche für folgende Vertragsstaaten : BE, CH, DE, FR, GB, LU, NL, SE**

1. Humane Hybridome, die zur Bildung von humanen monoklonalen Antikörpern fähig sind, welche humane neoplastische Zellen von normalen humanen Zellen unterscheiden, worin dieses humane Hybridom durch eine Fusion eines B-Lymphozyten aus einer Lymphknotendrainage eines menschlichen Wirts, der ein Neoplasma aufweist, mit einem Fusionspartner für humane Zellen erhalten werden kann, worin dieser Fusionspartner eine humane immortalisierte B-Zelle ist, die ihrerseits keine Immunglobuline sekretiert, oder hiervon abstammende humane Hybridome.

**2.** Humane Hybridome CLNH5 oder CLNH11 nach Anspruch 1.

**3.** Humane monoklonale Antikörper, die von humanen Hybridomen nach einem der Ansprüche 1 oder 2 erhalten wurden.

**4.** Humane monoklonale Antikörper nach Anspruch 3, worin die neoplastischen Zellen Zellen solider Tumoren sind.

**5.** Humane monoklonale Antikörper nach Anspruch 3, worin die neoplastischen Zellen Prostatakarzinomzellen, kleinzellige Lungenkarzinomzellen, Zervixkarzinomzellen, Kolonkarzinomzellen oder Melanomzellen sind.

**6.** Humane monoklonale Antikörper nach Anspruch 3, worin die neoplastischen Zellen Zervixkarzinomzellen sind.

**7.** Humane monoklonale Antikörper nach einem der Ansprüche 3 bis 6 oder Fragmente hiervon, die mit einer Markierung markiert sind, die ein detektierbares Signal liefern kann.

**8.** Humane monoklonale Antikörper nach Anspruch 7 oder Fragmente hiervon, worin diese Markierung ein Radionuklid ist.

**9.** Humane monoklonale Antikörper nach einem der Ansprüche 3 bis 6 oder Fragmente hiervon, die mit einem Toxin markiert sind.

**10.** Verwendung der monoklonalen Antikörper oder der Fragmente hiervon nach einem der Ansprüche 3 bis 9, um das Vorkommen eines Neoplasmas zu bestimmen, welche umfaßt:
- Zusammenbringen einer Probe eines Wirts, bei dem ein Neoplasma vermutet wird, mit den monoklonalen Antikörpern oder Fragmenten hiervon und
- Detektion der Bindung dieser monoklonalen Antikörper oder Fragmente hiervon.

**11.** Verwendung der monoklonalen Antikörper nach Anspruch 10, worin diese Probe ein Wirtsgewebe ist.

**12.** Verwendung der monoklonalen Antikörper nach Anspruch 10 oder 11, worin dieses Neoplasma ein solider Tumor ist.

**13.** Verwendung der monoklonalen Antikörper nach Anspruch 12, worin dieser solide Tumor ein Zervixkarzinom, Prostatatumor, Kolonkarzinom, Lungenkrebs, Brustkrebs oder Melanom ist.

**14.** Verfahren zur Herstellung von humanen monoklonalen Antikörpern nach einem der Ansprüche 3 bis 9, das die Anzucht eines Hybridoms nach Anspruch 1 oder 2 und die Gewinnung der von diesem Hybridom gebildeten Antikörper, und falls gewünscht, die Markierung der Antikörper umfaßt.

**15.** Humane monoklonale Antikörper oder Fragmente hiervon nach einem der Ansprüche 3 bis 9 zur Verwendung in einem Behandlungsverfahren des menschlichen Körpers bei einer Therapie.

**Patentansprüche für folgenden Vertragsstaat : AT**

**1.** Verfahren zur Herstellung von humanen monoklonalen Antikörpern, die humane neoplastische Zellen von normalen humanen Zellen unterscheiden können, das die Anzucht eines Hybridoms und die Gewinnung der durch das Hybridom gebildeten Antikörper umfaßt, worin dieses Hybridom durch eine Fusion eines B-Lymphozyten aus einer Lymphknotendrainage eines menschlichen Wirts, der ein Neoplasma aufweist, mit einem Fusionspartner für humane Zellen erhalten werden kann, worin dieser Fusionspartner eine humane immortalisierte B-Zelle ist, die ihrerseits keine Immunglobuline sekretiert.

**2.** Verfahren nach Anspruch 1, worin das Hybridom CLNH5 oder CLNH11 ist.

**3.** Verfahren nach Anspruch 1 oder 2, worin die neoplastischen Zellen Zellen solider Tumoren sind.

**4.** Verfahren nach Anspruch 1 oder 2, worin die neoplastischen Zellen Prostatakarzinomzellen, kleinzellige Lungenkarzinomzellen, Zervixkarzinomzellen, Kolonkarzinomzellen oder Melanomzellen sind.

5.  Verfahren nach Anspruch 1 oder 2, worin die neoplastischen Zellen Zervixkarzinomzellen sind.

6.  Verfahren nach einem der vorangehenden Ansprüche, worin die Antikörper oder Fragmente hiervon mit einer Markierung markiert sind, die ein detektierbares Signal liefern kann.

7.  Verfahren nach Anspruch 6, worin die Markierung ein Radionuklid ist.

8.  Verfahren nach Anspruch 6, worin die Markierung ein Toxin ist.

9.  Verwendung der monoklonalen Antikörper oder der Fragmente hiervon nach einem der Ansprüche 3 bis 8, um das Vorkommen eines Neplasmas zu bestimmen, welche umfaßt:
    - Zusammenbringen einer Probe von einem Wirt, bei dem ein Neoplasma vermutet wird, mit den monoklonalen Antikörpern oder Fragmenten hiervon und
    - Detektion der Bindung dieser monoklonalen Antikörper oder Fragmente hiervon.

10. Verwendung der monoklonalen Antikörper nach Anspruch 9, worin diese Probe ein Wirtsgewebe ist.

11. Verwendung der monoklonalen Antikörper nach Anspruch 9 oder 10, worin dieses Neoplasma ein solider Tumor ist.

12. Verwendung der monoklonalen Antikörper nach Anspruch 11, worin dieser solide Tumor ein Zervixkarzinom, Prostatatumor, Kolonkarzinom, Lungenkrebs, Brustkrebs oder Melanom ist.

13. Humane monoklonale Antikörper oder Fragmente hiervon, die nach einem Verfahren der Ansprüche 1 bis 8 erhalten wurden, zur Verwendung in einem Behandlungsverfahren des menschlichen Körpers bei einer Therapie.


## Revendications

### Revendications pour les Etats contractants suivants : BE, CH, DE, FR, GB, LU, NL, SE

1.  Hybridomes humains capables de produire des anticorps monoclonaux humains qui distinguent les cellules néoplasiques humaines des cellules humaines normales, un tel hybridome humain pouvant être obtenu par fusion d'un lymphocyte B, issu d'un ganglion lymphatique de drainage d'un hôte humain atteint d'une néoplasie, avec un partenaire de fusion pour cellules humaines, ce partenaire de fusion étant un lymphocyte B humain immortalisé qui ne sécrète pas lui-même d'immunoglobulines, ou hybridomes humains qui en dérivent.

2.  Hybridomes humains CLNH5 ou CLNH11 conformes à la revendication 1.

3.  Anticorps monoclonaux humains obtenus à partir d'hybridomes humains conformes à l'une des revendications 1 et 2.

4.  Anticorps monoclonaux humains conformes à la revendication 3, pour lesquels les cellules néoplasiques sont des cellules de tumeur solide.

5.  Anticorps monoclonaux humains conformes à la revendication 3, pour lesquels les cellules néoplasiques sont des cellules de cancer de la prostate, des cellules de cancer pulmonaire à petites cellules, des cellules de cancer du col de l'utérus, des cellules de cancer du côlon ou des cellules de mélanome.

6.  Anticorps monoclonaux humains conformes à la revendication 3, pour lesquels les cellules néoplasiques sont des cellules de cancer du col de l'utérus.

7.  Anticorps monoclonaux humains conformes à l'une des revendications 3 à 6 ou fragments de tels anticorps, marqués avec un marqueur capable de fournir un signal détectable.

8.  Anticorps monoclonaux humains conformes à la revendication 7 ou fragments de tels anticorps, dans lesquels ledit marqueur est un radionucléide.

9. Anticorps monoclonaux humains conformes à l'une des revendications 3 à 6 ou fragments de tels anticorps, marqués avec une toxine.

10. Emploi d'anticorps monoclonaux ou de fragments de tels anticorps, conformes à l'une des revendications 3 à 9, pour déterminer la présence d'un néoplasme, ledit emploi comportant :
   - le fait de mettre en présence de tels anticorps monoclonaux ou de fragments de tels anticorps un échantillon provenant d'un hôte chez lequel on soupçonne la présence d'un néoplasme, et
   - le fait de détecter la fixation de ces anticorps monoclonaux ou de ces fragments.

11. Emploi d'anticorps monoclonaux, conforme à la revendication 10, pour lequel ledit échantillon est un tissu de l'hôte.

12. Emploi d'anticorps monoclonaux, conforme à la revendication 10 ou 11, pour lequel ledit néoplasme est une tumeur solide.

13. Emploi d'anticorps monoclonaux, conforme à la revendication 12, pour lequel ladite tumeur solide est un cancer du col de l'utérus, une tumeur de la prostate, un cancer du côlon, un cancer pulmonaire, un cancer du sein ou un mélanome.

14. Procédé de production d'anticorps monoclonaux humains conformes à l'une des revendications 3 à 9, qui comporte le fait de cultiver un hybridome conforme à la revendication 1 ou 2 et le fait de récupérer les anticorps produits par cet hybridome, et si on le souhaite, le fait de marquer ces anticorps.

15. Anticorps monoclonaux humains ou fragments de tels anticorps, conformes à l'une des revendications 3 à 9, destinés à être employés dans un procédé de traitement thérapeutique du corps humain.

**Revendications pour l'Etat contractant suivant : AT**

1. Procédé de production d'anticorps monoclonaux humains qui peuvent distinguer les cellules néoplasiques humaines des cellules humaines normales, qui comporte le fait de cultiver un hybridome et le fait de récupérer les anticorps produits par cet hybridome, ledit hybridome pouvant être obtenu par fusion d'un lymphocyte B, issu d'un ganglion lymphatique de drainage d'un hôte humain atteint d'une néoplasie, avec un partenaire de fusion pour cellules humaines, ce partenaire de fusion étant un lymphocyte B humain immortalisé qui ne sécrète pas lui-même d'immunoglobulines.

2. Procédé conforme à la revendication 1, dans lequel l'hybridome est CLNH5 ou CLNH11.

3. Procédé conforme à la revendication 1 ou 2, pour lequel les cellules néoplasiques sont des cellules de tumeur solide.

4. Procédé conforme à la revendication 1 ou 2, pour lequel les cellules néoplasiques sont des cellules de cancer de la prostate, des cellules de cancer pulmonaire à petites cellules, des cellules de cancer du col de l'utérus, des cellules de cancer du côlon ou des cellules de mélanome.

5. Procédé conforme à la revendication 1 ou 2, pour lequel les cellules néoplasiques sont des cellules de cancer du col de l'utérus.

6. Procédé conforme à l'une des revendications précédentes, dans lequel lesdits anticorps ou des fragments de ces anticorps sont marqués avec un marqueur capable de fournir un signal détectable.

7. Procédé conforme à la revendication 6, dans lequel ledit marqueur est un radionucléide.

8. Procédé conforme à la revendication 6, dans lequel ledit marqueur est une toxine.

9. Emploi d'anticorps monoclonaux ou de fragments de tels anticorps, produits selon l'une des revendications 3 à 8, pour déterminer la présence d'un néoplasme, ledit emploi comportant :
   - le fait de mettre en présence de tels anticorps monoclonaux ou de fragments de tels anticorps un échantillon provenant d'un hôte chez lequel on soupçonne la présence d'un néoplasme, et

- le fait de détecter la fixation de ces anticorps monoclonaux ou de ces fragments.

10. Emploi d'anticorps monoclonaux, conforme à la revendication 9, pour lequel ledit échantillon est un tissu de l'hôte.

11. Emploi d'anticorps monoclonaux, conforme à la revendication 9 ou 10, pour lequel ledit néoplasme est une tumeur solide.

12. Emploi d'anticorps monoclonaux, conforme à la revendication 11, pour lequel ladite tumeur solide est un cancer du col de l'utérus, une tumeur de la prostate, un cancer du côlon, un cancer pulmonaire, un cancer du sein ou un mélanome.

13. Anticorps monoclonaux humains ou fragments de tels anticorps, obtenus selon un procédé conforme à l'une des revendications 1 à 8, destinés à être employés dans un procédé de traitement thérapeutique du corps humain.